# EUROPEAN PATENT APPLICATION

(11) **EP 2 873 737 A1**
(43) Date of publication of application: **20.05.2015**
(21) Application number: 13192748.5
(22) Date of filing: 13.11.2013
(51) Int. Cl.: C12Q 1/68

(54) **A method and a kit suitable for determining that a human subject has or is at risk of developing type 1 diabetes mellitus**

(71) Applicant: Institute of Biology of the University of Latvia, 2169 Salaspils (LV); University of Latvia, 1586 Riga (LV)
(72) Inventor: Sjakste, Tatjana, 1082 Riga (LV); Paramonova, Natalia, 1063 Riga (LV); Sjakste, Nikolajs, 1082 Riga (LV)
(74) Representative: Fortuna, Jevgenijs

(57) **Abstract**

The present invention relates to a methods and kits for determining that a human subject has or is at risk of developing type 1 *diabetes mellitus* disease. The method according to the invention comprising: detecting, in a genomic DNA present in an isolated sample of biological material removed from the subject, the rs71928782 locus heterozygote genotype formed by (TG)₁₉ (SEQ ID No 1) and (TG)₂₃ (SEQ ID No 2), or the rs57727346 locus heterozygote genotype formed by (AC)₂₃ (SEQ ID No 3) and (AC)₂₅ (SEQ ID No 4), or the rs10647945 heterozygote genotype formed by (AC)₂₂ (SEQ ID No 5) and (AC)₂₅ (SEQ ID No 6) or their combinations, wherein presence of said genotypes indicates the presence of, or a risk of developing, type 1 *diabetes mellitus* disease in the subject. The kit for the detecting, diagnosing, prediction, prognosis, staging or monitoring type 1 *diabetes mellitus,* comprises DNA amplified fragment having nucleotide sequence SEQ ID No. 1 and SEQ ID No. 2, and/or DNA amplified fragment having nucleotide sequence SEQ ID No 3 and SEQ ID No 4, and/or DNA amplified fragment having nucleotide sequence SEQ ID No 5 and SEQ ID No 6. The kit may comprise synthetic primers of sequences SEQ ID No. 7 and SEQ ID No. 8, and/or SEQ ID No. 9 and SEQ ID No. 10, and/or SEQ ID No. 11 and SEQ ID No. 12.

SEQUENCE LISTING

<110> University of Latvia
      Genomics and Bioinformatics, Institute of Biology of the
      University of Latvia

<120> A method and a kit suitable for determining that a human subject has
or is at risk of developing type 1 diabetes mellitus

<160> 12

<170> BiSSAP 1.2

<210> 1
<211> 183
<212> DNA
<213> Homo sapiens

<220>
<221> source
<222> 1..183
<223> /mol_type="unassigned DNA"
      /note="rs34580276 (HSMS006) allele (TG)19"
      /organism="Homo sapiens"

<400> 1
ctaattgact tgcagttgct ggatgtgtgt gtgtgtgtgt gtgtgtgtgt gtgtgtgtgt      60

gttcttactc actaagtgac taactcccac acatcagcct tatagcttcc atgattcaaa     120

tccatactga gtgcttcgta taatcagctc atttaatcct cccagcagcc ctgtgaggca     180

gtt                                                                   183


<210> 2
<211> 191
<212> DNA
<213> Homo sapiens

<220>
<221> source
<222> 1..191
<223> /mol_type="unassigned DNA"
      /note="rs34580276 (HSMS006) allele (TG)23"
      /organism="Homo sapiens"

<400> 2
ctaattgact tgcagttgct ggatgtgtgt gtgtgtgtgt gtgtgtgtgt gtgtgtgtgt      60

gtgtgtgtgt tcttactcac taagtgacta actcccacac atcagcctta tagcttccat     120

gattcaaatc catactgagt gcttcgtata atcagctcat ttaatcctcc cagcagccct     180

gtgaggcagt t                                                          191


<210> 3
<211> 132
<212> DNA
<213> Homo sapiens

<220>

<221> source
<222> 1..132
<223> /mol_type="unassigned DNA"
      /note="rs57727346 (HSMS701) allele (AC)23"
      /organism="Homo sapiens"

<400> 3
tccatagcac ttatctgaca tgctacacac acacatacac acacacacac acacacacac      60

acacacacac agagctttta ctatatatct tttctctgta tagagtgaaa gcaccacaag     120

ggtagggatt tt                                                         132


<210> 4
<211> 136
<212> DNA
<213> Homo sapiens

<220>
<221> source
<222> 1..136
<223> /mol_type="unassigned DNA"
      /note="rs57727346 (HSMS701) allele (AC)25"
      /organism="Homo sapiens"

<400> 4
tccatagcac ttatctgaca tgctacacac acacatacac acacacacac acacacacac      60

acacacacac acacagagct tttactatat atcttttctc tgtatagagt gaaagcacca     120

caagggtagg gatttt                                                     136


<210> 5
<211> 149
<212> DNA
<213> Homo sapiens

<220>
<221> source
<222> 1..149
<223> /mol_type="unassigned DNA"
      /note="rs10647945 (HSMS801) allele (AC)22"
      /organism="Homo sapiens"

<400> 5
atgcagtatg ccaccaactg tgtaaagaca ggaaaagaga acacacacac acacacacac      60

acacacacac acacacacac acacttgctt gttgatgtct agtctctaga aggctactga     120

agaaatggat aatactggtt gcctctcgg                                       149


<210> 6
<211> 155
<212> DNA
<213> Homo sapiens

<220>
<221> source
<222> 1..155
<223> /mol_type="unassigned DNA"

      /note="rs10647945 (HSMS801) allele (AC)25"
      /organism="Homo sapiens"

<400> 6
atgcagtatg ccaccaactg tgtaaagaca ggaaaagaga acacacacac acacacacac      60

acacacacac acacacacac acacacacac ttgcttgttg atgtctagtc tctagaaggc     120

tactgaagaa atggataata ctggttgcct ctcgg                                155


<210> 7
<211> 22
<212> DNA
<213> Artificial Sequence Synthetic Primer

<220>
<221> source
<222> 1..22
<223> /mol_type="unassigned DNA"
      /note="Description of Artificial Sequence: Synthetic Forward
      Primer for the rs34580276"
      /organism="Artificial Sequence Synthetic Primer"

<400> 7
ctaattgact tgcagttgct gg                                               22


<210> 8
<211> 18
<212> DNA
<213> Artificial Sequence Synthetic Primer

<220>
<221> source
<222> 1..18
<223> /mol_type="unassigned DNA"
      /note="Description of Artificial Sequence: Synthetic Reversal
      Primer for the rs34580276"
      /organism="Artificial Sequence Synthetic Primer"

<400> 8
aactgcctca cagggctg                                                    18


<210> 9
<211> 23
<212> DNA
<213> Artificial Sequence Synthetic Primer

<220>
<221> source
<222> 1..23
<223> /mol_type="unassigned DNA"
      /note="Description of Artificial Sequence: Synthetic Forward
      Primer for the rs57727346"
      /organism="Artificial Sequence Synthetic Primer"

<400> 9
tccatagcac ttatctgaca tgc                                              23


<210> 10

<211> 21
<212> DNA
<213> Artificial Sequence Synthetic Primer

<220>
<221> source
<222> 1..21
<223> /mol_type="unassigned DNA"
      /note="Description of Artificial Sequence: Synthetic Reversal
      Primer for the rs57727346"
      /organism="Artificial Sequence Synthetic Primer"

<400> 10
aaaatcccta cccttgtggt g                                                21


<210> 11
<211> 21
<212> DNA
<213> Artificial Sequence Synthetic Primer

<220>
<221> source
<222> 1..21
<223> /mol_type="unassigned DNA"
      /note="Description of Artificial Sequence: Synthetic Forward
      Primer for the rs10647945"
      /organism="Artificial Sequence Synthetic Primer"

<400> 11
atgcagtatg ccaccaactg t                                                21


<210> 12
<211> 22
<212> DNA
<213> Artificial Sequence Synthetic Primer

<220>
<221> source
<222> 1..22
<223> /mol_type="unassigned DNA"
      /note="Description of Artificial Sequence: Synthetic Reversal
      Primer for the rs10647945"
      /organism="Artificial Sequence Synthetic Primer"

<400> 12
ccgagaggca accagtatta tc                                               22

## Description

*The research was supported by the European Regional Development Foundation funding under the agreement No. 2010*/*0315*/*2DP*/*2.1.1.1.0*/*10*/*APIA*/*VlAA*/*026.*

### Technical field

The present invention relates to a method for determining type 1 *diabetes mellitus* (T1DM) disease, which comprises detecting gene polymorphisms existing in a *KIAA0391-PSMA6* gene cluster, biomarkers associated with type 1 *diabetes mellitus,* an oligonucleotide and amplicons used in the method, a kit for diagnosing type 1 *diabetes mellitus* comprising the oligonucleotide and amplicons and a use for detection, prediction, prognosis, prediction of therapy outcome, and monitoring of the progression of type 1 *diabetes mellitus.*

### Background Art

Diabetes mellitus (DM) and its complications cause numerous health and social problems throughout the world. Number of DM patients constantly increases. Diabetes, characterized by persistent elevation of blood glucose levels (hyperglycaemia), occurs due to inadequate production of insulin (type 1 diabetes; T1DM), or resistance to endogenous insulin usually associated with the metabolic syndrome and obesity (type 2 diabetes, T2DM). Despite intensive glycaemic control, individuals with T1DM and T2DM are predisposed to developing vascular complications (reviewed in Sharma, Khanna, 2013). DM and its complications are common in Latvia: in 2010 72654 DM patients were registered, 3891 with T1DM and 68217 with T2DM. Etiologically T1DM is an autoimmune disease both environmental and hereditary factors being important for susceptibility to it. Several studies have demonstrated a fundamental role for the HLA in the susceptibility of, or protection to T1DM. While HLA remains the strongest genetic risk factor, a number of novel gene variants associated with T1DM have been found through genome-wide studies, some of which have been linked to suspected environmental risk factors. However, jointly non-HLA risk alleles described up till now confer only a small additional risk compared to the effect of *HLADR, HLADQ.* (Nokoff, Rewers, 2013). It should be mentioned that some genetic predisposition factors apparently are not spotted in GWAS studies, although sufficient association is revealed in studies with individual genes.

An exceptional biological role of the ubiquitin proteasome system (UPS) in the antigen processing and immune response suggested by Kloetzel (2001), was greatly supported experimentally during last decade. It is well known nowadays that complex and dynamic UPS system is represented by unique in place and time organization of multiple subunits for realization of any physiological process and in response to additional internal or external stimuli. Numerous data indicate possible involvement of the ubiquitin-proteasome system in regulation of insulin action and pathogenesis of *diabetes mellitus.* Induction of *diabetes mellitus* causes redistribution of the proteasome subtypes (Merforth et al., 2003). Proteasome activities are involved in the first steps of the T1DM pathogenesis. The disease results from autoimmune destruction of the insulin producing pancreatic β-cells. The immunoproteasome, a version of the proteasome that collaborates with the 11S/PA28 activator to generate immunogenic peptides for presentation by MHC class I molecules implicated in the onset of the disease. Interferon β(IFNβ), mediator of the early antiviral defenses, stimulates expression of the immunoproteasome and the 11S/PA28 activator; however expression of regular proteolytic subunits remains unchanged. As a result, immunoproteasomes had stochastic combinations of immune and regular proteolytic sites, an arrangement that would likely increase the probability with which unique immunogenic peptides are produced (Freudenburg et al., 2013). The ubiquitin proteasome system is important also to maintain pancreatic beta cell function. Inhibition of the proteasome significantly reduces glucose-induced insulin secretion. Key regulators of the stimulus-secretion cascade seem to be affected by protein misfolding. Glucokinase misfolding and aggregation is observed in case of a diminished capacity of the ubiquitin proteasome system in pancreatic beta cells (Hofmeister-Brix et al., 2013). Ubiquitin-proteasome system is also involved in pathogenesis of T1DM complications. The disease is not simply glucose deregulation; it is a chronic inflammatory disease that affects nearly every biological process, including protein metabolism. Diabetes is associated with disturbances in muscle protein metabolism that results in decreased muscle mass and in some cases, loss in the activities of daily living, decreased productivity and diminished quality of life. In type 1 diabetes, muscle wasting essentially results from insulin deficiency and this induces of genes involved in the ubiquitin proteasome pathway (Workeneh, Bajaj, 2013). Ubiquitination is involved in pathogenesis of diabetic nephropathy on the level of epigenetic regulation of genome expression. The high glucose may induce H2A ubiquitination and reduce H2B ubiquitination in kidney cells of diabetics (Gao et al., 2013). Development of diabetic retinopathy is also a proteasome-mediated process. High glucose has direct biological effects on cellular proteasome function, the level of total ubiquitin-conjugated proteins are increased in retina cells, proteasome chymotrypsin-like peptidase activity is decreased in perivascular supporting cells (Aghdam et al., 2013). Inhibition of proteasome activity reduces the manifestation of DM in animals (Petrovic et al., 2004).

In preceding polymorphism studies the inventors of present invention have focused their attention on the long arm of the human Chromosome 14 as it contains a cluster of proteasomal genes including the *PSMB5, PSME1, PSME2* (all in 14q11.2), *PSMA6* (14q13), *PSMC6* (14q22.1), *PSMA3* (14q23), *PSMA3P* (14q23.1) and *PSMC1* (14q32.11) genes. Indeed, some studies on the search for susceptibility genes for diabetes mellitus have indicated the probable involvement of several loci on the long arm of the Chromosome 14 in hereditary predisposition to the disease (Permutt *et al.,* 2001; Silander *et al.,* 2004; *Hsueh et al.,* 2003). In previous studies the inventors have detected Type 2 *diabetes mellitus* association with microsatellite markers of the region (rs63749745, rs71444202 and rs34580276; Sjakste et al, 2007), *PSMA6* c.-8C>G (rs1048990) and *PSMA6* c.-109-1A/c.-8G (rs2277460/rs1048990) haplotype (Sjakste et al., 2007b). The rs63749745 showed high level of association with Graves' disease (Sjakste et al., 2004), the rs71928782, rs5807818, rs71444202 and rs345802276 and SNPs rs2277460 (*PSMA6*), rs2295826 and rs2295827 (*PSMC6*) and rs2348071 (*PSMA3*) were found to be in association with juvenile idiopathic arthritis in children (Sjakste et al., 2010 and Sjakste et al., 2013 submitted). The inventors' results were replicated in other populations (Wang et al., 2013; Bennett et al., 2008; Barbieri et al., 2008; Liu et al., 2009; Hinohara et al., 2009; Alsmadi et al., 2009; Ikeda et al., 2010; Heckman et al., 2013; Liu et al., 2012), a patent for using markers of the region for diagnostics of inflammatory diseases has been issued (Tanaka et al., 2012).

Microsatellites (MS) are repeating sequences of 2-6 base pairs of DNA (Turnpenny, Ellard, 2005) They are used as molecular markers in genetics, for kinship, population and other studies. They can also be used for studies of gene duplication or deletion, marker assisted selection, and fingerprinting. Microsatellites are distributed throughout the genome (King, 2012). Being variable genetic elements, microsatellites provide a potent tool for individual characterization of genomes. Variability is generated due to replication slippage caused by mismatches between DNA strands while being replicated during meiosis (Tautz, Schlotter, 1994), the event can occur once per 1,000 generations (Weber, Wong 1993). These slippages are much more common compared to point mutations (Jarne, Lagoda, 1996). Microsatellite repeats are able to mutagenize the genomes of human individuals and are therefore poised to dynamically alter the human genomic landscape across generations (Grandi, An, 2013). Utility of microsatellites demonstrates the study comprising 2,058 germline changes discovered by analyzing 85,289 Icelanders at 2,477 microsatellites. The paternal-to-maternal mutation rate ratio is 3.3, and the rate in fathers doubles from age 20 to 58, whereas there is no association with age in mothers. Longer microsatellite alleles are more mutagenic and tend to decrease in length, whereas the opposite is seen for shorter alleles (Sun et al., 2012).

Microsatellites remain highly informative and useful measures of genomic variation for linkage and association studies despite the fact that general preference is given to SNPs. Their continued advantage in complementing SNPs lies in their greater allelic diversity than biallelic SNPs as well as in their population history, in which single-step expansion or contraction of the tandem repeat on the background of ancestral SNP haplotypes can break up common haplotypes, leading to greater haplotype diversity (Gulcher, 2013).

Although mostly used as structural genetic markers microsatellites perform several functions in genome which are still far from being completely understood. However, in recent years it has become clear that MS and their flanking regions are functionally active participants of gene and genome function. MS are known to form nuclear matrix anchorage sites (Boulikas, 1993), tissue specific matrix attachment sites (Lenartowski, Goc, 2002), binding sites with vimentin and glial fibrillary acidic protein (Tolstonog et al., 2005) and give rise to complex DNA spatial structures of extreme functional significance (Tolstonog et al., 2005; Li et al., 2003; Rothenburg et al., 2001). MS appear to be important components of insulators (Filippova et al., 2001), silencers (Rothenburg et al., 2001) and enhancers (Bassuny et al., 2003). MSs are also involved in regulation of alternative splicing (Hui et al., 2005), mRNA stability (Lee et al., 2004), recombination and repair (Wang & Vasquez, 2007; Chin et al., 2007). The sequence motif of the intronic MS could be one of the main features that defines intron haplotype (Sjakste & Zhuk, 2006; Van Wormhoudt & Sellos, 2003) and could be linked with trait responsible single nucleotide polymorphism (SNP) of gene coding region (cSNP) in neighbouring exons (Sjakste & Zhuk, 2006). Microsatellites can determine activity of the upstream gene regulation elements like Locus Control Region of the bet-globin gene domain. (AT)₈N₁₂GT(AT)₇ configuration of microsatellite found in the Hypersensitive site of the structure is associated with a special form of sickle cells - Tunisian βs chromosomes (Ben Mustapha et al., 2012). Length changes of microsatellites within promoters and other cis-regulatory regions can also change level of gene expression; they are linked to abundant variations in cis-regulatory control regions in the human genome (Rockman, Wray, 2002). For example, a CA-repeat microsatellite in insulin-like growth factor 1 (IGF1) promoter is associated with the level of this growth factor. It turned out that intensity of the gene transcription is regulated by interaction of several SNPs and the microsatellite generating haplotypes with lower or higher levels of the gene transcription (Chen et al., 2013a). Promoter microsatellites tend to be G/C rich, thee are found often at the start of the genes and are probably associated with regulatory elements as CpG islands, G-quadruplexes (G4) and untranslated regulatory regions. Many promoter microsatellites have the potential to affect human phenotypes by generating mutations in regulatory elements, which may ultimately result in disease (Sawaya et al., 2013). A CpG-CA repeat within the human endothelin-converting enzyme-1 (ECE-1) promoter is highly polymorphic, harbours transcriptional start sites, it is able to recruit the transcription factors and poly(ADP-ribose) polymerase-1 and splicing factors, and is functional regarding haplotype-specific promoter activity. Overall CpG-CA repeat composition of Alzheimer disease patients and controls is distinct (Li et al., 2012). Microsatellites localized in the 3'-UTR regions UTR may affect the final mRNA stability, the localisation, the export from the nucleus and the translation efficiency. The androgen receptor CAG repeat polymorphism (AR CAG) affects receptor transcriptional activity (the shorter repeats the more sensitive AR) and is associated with androgenic parameters and obesity (Stanworth et al., 2011). The conserved regulatory sequences within 3' UTRs and the specific elements binding to them enable gene expression control at the posttranscriptional level and all these processes reflect the actual state of the cell (Michalova et al., 2013) .

Microsatellites within introns also influence phenotype, through means that are not currently understood, this is the cause of numerous associations of microsatellite repeat polymorphisms with human diseases. For example, a GAA triplet expansion in the first intron of the X25 gene appears to interfere with transcription, and causes Friedreich Ataxia (Bidichandani et al., 1998). Expansions of microsatellite DNA repeats contribute to the inheritance of nearly 30 developmental and neurological disorders (Kim, Mirkin, 2013). Restricting our analysis to diabetes mellitus and its complications and predisposing diseases numerous examples of association can be demonstrated.

A length polymorphism of GT repeats in the promoter region of the human heme oxygenase-1 (HO-1) gene modulates its gene transcription (Hu et al., 2013). A number of studies have associated human HO-1 gene promoter polymorphisms with risk for vascular diseases (Wu et al., 2010). Persons carrying longer (GT)ₙ repeats in the *HMOX1* gene (L allele) promoter may have a higher risk of type 2 diabetes (Bao et al., 2010). In functional analyses, HO-1 expression level was significantly reduced in persons with impaired glucose regulation and T2DM carrying the L/L (GT)ₙ genotype compared with persons with the S/S genotype (Song et al., 2009). The same microsatellite is associated with susceptibility to cardiovascular complications of the disease. Patients with longer lengths of GT repeats in the heme oxygenase-1 gene promoter exhibit higher inflammation and oxidative stress. These patients have higher risk of long-term cardiovascular events and mortality (Chen et al., 2013b).

Subjects having more CA repeats in the first intron of the type 2 11β-hydroxysteroid dehydrogenase gene (*HSD11B2*) are susceptible to developing abnormal glucose tolerance (Mune et al., 2013). Carnosinase D18S880 microsatellite polymorphism, especially five trinucleotide repeat homozygotes, is associated with diabetic nephropathy susceptibility (Zhu et al., 2013). The aldose reductase (*AKRIB1*) gene harbours a microsatellite significantly associated with diabetic retinopathy. The z-2 microsatellite was found to confer risk in type 1 and type 2 diabetes and z+2 to confer protection against diabetic retinopathy in type 2 diabetes regardless of ethnicity (Abhary et al., 2009).

Cardiovascular diseases being predisposing to diabetes mellitus or arising as its complications might be also associated with certain microasatellite alleles. A repeat polymorphism in the fourth intron of the *NOS3* gene is linked to hypertension (Jemaa 2009). Haplotypes comprising S allele of (CCTTT)ₙ repeat in *NOS2* gene are associated both with hypertension and responsiveness to antihypertensive drug therapy (Oliveira-Paula et al., 2013). Short allele of the same microsatellite might be associated with the abdominal aortic aneurysm (Gregorek et al., 2013). Shorter alleles of microsatellites in the 3' flanking region of leptin gene,coding for a protein hormone, mainly synthesized in adipocytes, that regulates the food intake and energy expenditure of the body, are significantly associated with hypertension (Akhter et al., 2012).

### Disclosure of Invention

It is an object of the present invention to provide a method for determining risk of type 1 *diabetes mellitus,* which involves identifying polymorphisms associated with type 1 *diabetes mellitus* and using the genes' microsatellite (simple sequence repeat) polymorphism, an oligonucleotide and amplicons that can be used for the method, a kit for diagnosing type 1 *diabetes mellitus,* and the like.

The present invention provides a technology for determining risk for development of type 1 *diabetes mellitus,* which comprises detecting of several microsatellite polymorphisms existing within and in the locus of proteasome subunit α type 6 gene (*PSMA6*) and *KIAA0391* gene on human Chromosome 14q13.

The repeats are amplified in PCR reaction, length of the fragments is determined electrophoretically. The determined ensemble of fragment lengths, describing allele and genotype distribution of the individual gives information on genotype pattern of the markers.

There is offered a method for *in vitro* determining that a human subject has or is at risk of developing type 1 *diabetes mellitus,* said method comprising: detecting, in a genomic DNA present in an isolated sample of biological material removed from the subject (e.g. body fluid, including blood, plasma, serum, synovial fluid, urine, feces, interstital fluid, lymphatic fluid, saliva, sudor, spinal fluid, lacrimal fluid; or another biological material or its fraction removed from the subject), the rs71928782 locus heterozygote genotype formed by (TG)₁₉ (SEQ ID No 1) and (TG)₂₃ (SEQ ID No 2), or the rs57727346 locus heterozygote genotype formed by (AC)₂₃ (SEQ ID No 3) and (AC)₂₅ (SEQ ID No 4), or the rs 10647945 heterozygote genotype formed by (AC)₂₂ (SEQ ID No 5) and (AC)₂₅ (SEQ ID No 6) or their combinations, wherein presence of said genotypes indicates the presence of, or a risk of developing, type 1 *diabetes mellitus* disease in the subject.

There is also offered a kit suitable for the detecting, diagnosing, prediction, prognosis, staging or monitoring type 1 *diabetes mellitus,* comprising DNA amplified fragment (amplicon) having nucleotide sequence SEQ ID No. 1 and DNA amplified fragment having nucleotide sequence SEQ ID No. 2, or DNA amplified fragment having nucleotide sequence SEQ ID No 3 and DNA amplified fragment having nucleotide sequence SEQ ID No 4, or DNA amplified fragment having nucleotide sequence SEQ ID No 5 and DNA amplified fragment having nucleotide sequence SEQ ID No 6, or combinations of pairs of sequences SEQ ID No. 1 - SEQ ID No. 2, SEQ ID No. 3 - SEQ ID No. 4 and SEQ ID No. 5 - SEQ ID No. 6. The kit may comprise synthetic primers of sequences SEQ ID No. 7 and SEQ ID No. 8, and/or SEQ ID No. 9 and SEQ ID No. 10, and/or SEQ ID No. 11 and SEQ ID No. 12. The offered kits may be used for the detecting, prediction, diagnosis, prognosis, staging or monitoring type 1 *diabetes mellitus.*

### Brief description of Figures

Fig. 1 - illustrates the HSMS microsatellite markers' location in the 14q13 300 Kb region (NC_000014.8: 35 500 KB...35 800 Kb);
Fig. 2 - shows summary on the rs34580276 locus variation;
Fig. 3 - shows summary on the rs57727346 locus variation;
Fig. 4 - shows summary on the rs 10647945 locus variation;
Fig. 5 - the rs34580276 chromatogram of amplified fragment (1), expected sequence of the amplified fragment according to chromatogram sizing (2), data verification by sequencing (3), recalculation of fragment size to repeat number (4), chromatogram of risk (TG)₁₉/(TG)₂₃ genotype (5) and alleles' sequence alignment of risk genotypes (6);
Fig. 6 - the rs57727346 chromatogram of amplified fragment (1), expected sequence of the amplified fragment according to chromatogram sizing (2), data verification by sequencing (3), recalculation of fragment size to repeat number (4), chromatogram of risk (AC)₂₃/(AC)₂₅ genotype (5) and alleles' sequence alignment of risk genotypes (6);
Fig. 7 - the rs10647945 chromatogram of amplified fragment (1), expected sequence of the amplified fragment according to chromatogram sizing (2), data verification by sequencing (3), recalculation of fragment size to repeat number (4), chromatogram of risk (AC)₂₂/(AC)₂₅ genotype (5) and alleles' sequence alignment of risk genotypes (6).

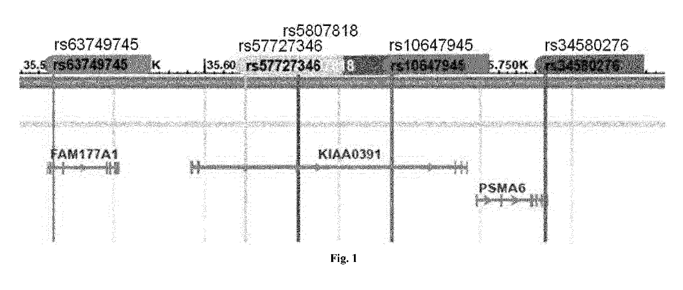

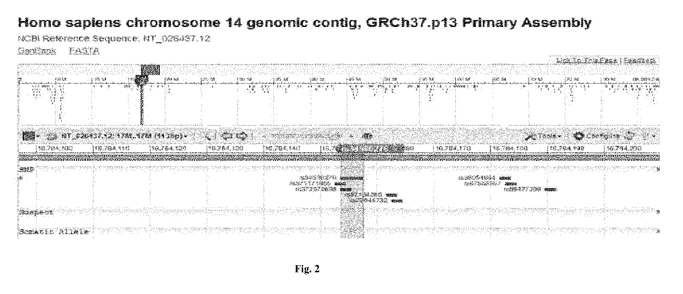

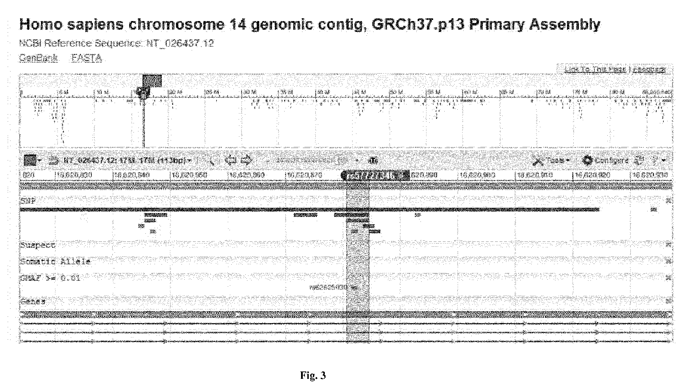

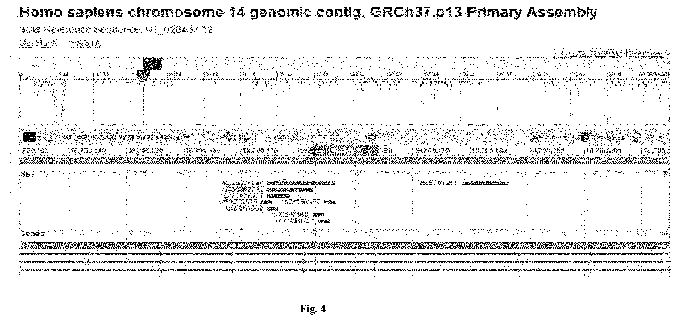

The inventors have performed a study of T1DM association with repeat number variation of intronic microsatellites localized in the 14q13 locus in the *FAM177A1* (rs63749745), KIAA0391 (rs57727346, rs5807818, and rs10647945) and *PSMA6* (rs34580276) genes. Localization of markers on Chromosome 14q13 region and in the mentioned genes is presented on Fig 1. Markers genetic diversity was previously studied in Latvian and Botnia human population (Sjakste et al, 2002; 2004; 2007; Sjakste et al., 2010). Inventors did not identify any association between T1DM and repeat number variation at the rs63749745 and rs5807818 loci (data are not present and will not be discussed further). The rs63749745, rs 10647945 and rs34580276 were found to be associated with T1DM. Tables 2, 3, 4, and 5 and Fig. 2, 3, and 4 provide detailed information on the rs63749745, rs10647945 and rs34580276 alternative marker identification numbers, HGVS names and motifs, sequences of primers used by inventors for genotyping, expected sizes of amplified fragments and their recalculation in number of repeats and genomic sequences encompassing the rs63749745, rs10647945 and rs34580276 microsatellites.

*Explanation of the terms used in the description and claims.*

By "biomarker" or "marker" a biochemical characteristic that can be used to diagnose, or to measure the progress of a disease or condition, or the effects of treatment of a disease or condition is meant. A biomarker can be, for example, the presence of a nucleic acid, protein, or antibody associated with the presence of diabetes or another disease in an individual. The present invention provides biomarkers for type 1 *diabetes mellitus* that are microsatellite repeat polymorphisms in a nucleic acid present in a sample of biological material removed from the subject. Microsatellites (MS) are repeating sequences of 2-6 base pairs of DNA like (TG)ₙ, (AC)ₙ, (CT)ₙ, (AT)ₙ or similar.

### Materials and methods

218 controls (138 women) were patients of the Riga Bikernieki Hospital without autoimmune and/or cardiovascular disorders, diabetes mellitus, or obesity. 200 DNA samples of the T1DM patients (103 women) were obtained from the Latvian Genome database collection. The study was approved by the Central Medical Ethics Commission of the Latvian Ministry of Health. Informed consent was obtained from all the study participants or their parents.

### DNA extraction and genotyping

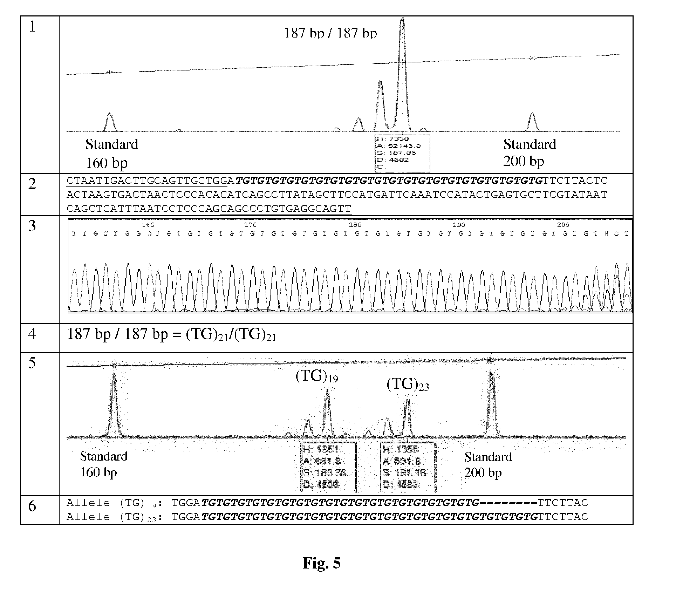

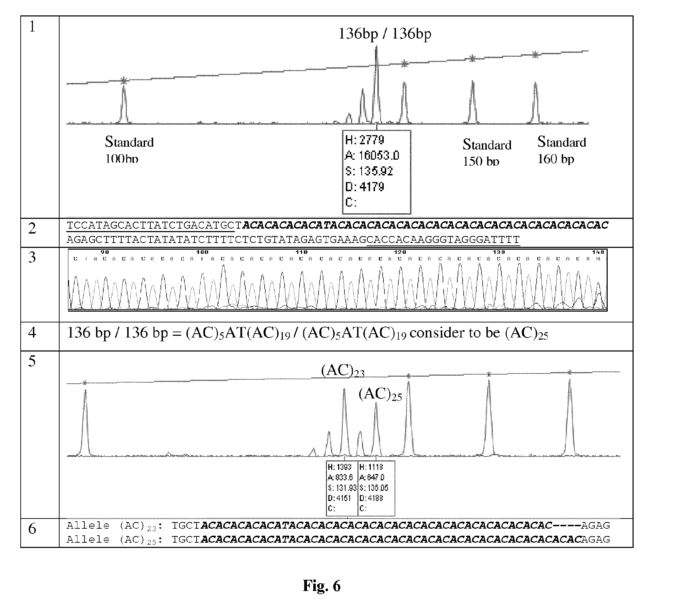

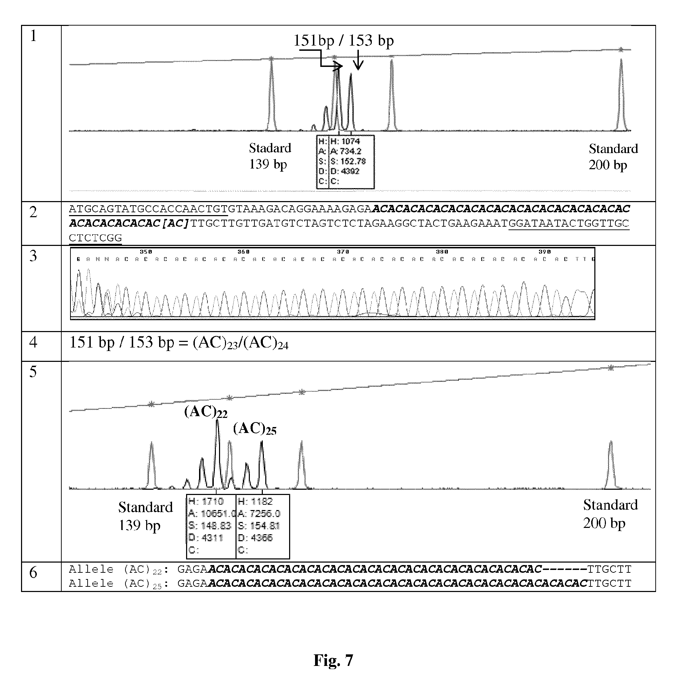

DNA was extracted using a kit for genomic DNA extraction from nucleated blood cells (Fermentas, Vilnius, Lithuania). Primer design was performed by the Primer 3.0 program using the 14q13.2 sequence information (NT_026437) at NCBI nucleotide service. PCR products (amplicons) for MS fragment sizing were amplified with HSMS primers as described (Sjakste et al. 2002, 2004, 2007, 2010). Primers for the rs34580276, rs57727346 and rs10647945 amplicons are indicated in the Table 2. PCR conditions were: 5 min at 94°C; 35 cycles of 30 seconds at 94°C, 30 seconds at 55°C, 30 seconds at 72°C; 10 min at 72°C; 4°C. DNA fragments were separated by capillary electrophoresis using the ABI Prism® 310 Genetic Analyzer (Applied Biosystems). Sizing of the PCR fragments was performed using ABI Prism Genotyper (v. 3.7, Applied Biosystems) or Peak Scanner (v.1.0, Applied Biosystems) software. The number of repeats in MS was deduced by comparison of the fragment sizing and sequencing data. Figs. 5, 6, and 7 illustrate strategy of the experiment and data management for the rs34580276, rs57727346 and rs10647945 respectively including the chromatograms of amplified fragment (Panels 1), expected sequence of the amplified fragments according to chromatogram sizing (Panels 2), data verification by sequencing (Panels 3), recalculation of fragment size to repeat number (Panels 4), chromatograms of risk genotypes (Panels 5) and alleles' sequence alignment of risk genotypes (Panels 6).

### Data analysis

Numbers and frequencies of alleles and genotypes were estimated by direct counting. The difference in allele and genotype frequencies (*Pa*) was analyzed by χ² test. Odds ratio (OR), relative risk (RR) and 95% confidence intervals (CI) were calculated by the MedCalc 3000 program (http://medicalc3000.com/OddsRatio.htm). Only OR > 2 was considered to be clinically significant.

### Results

Genotyping call rate was 100% for all three markers resulting in identification of 3-locus genotype per each individual of the study. All three markers are of high level of genetic diversity. The rs34580276 (HSMS006) locus was represented by 28 and 30 genotypes in controls and T1DM patients respectively (Table 6); only 9 and 8 genotypes showed frequency more than 5% in controls and T1DM group respectively. The rs57727346 (HSMS701) locus was represented by 27 and 23 genotypes in controls and T1DM patients respectively (Table 7); only 9 and 7 genotypes showed frequency more than 5% in controls and T1DM group respectively. The 10647945 (HSMA801) showed highest level of genetic diversity having 50 and 40 genotypes in control and T1DM groups respectively (Table 8) with only 6 and 5 genotypes showing frequency more than 5% in controls and T1DM group respectively. The genotype rs34580276 (TG)₁₉/(TG)₂₃ (frequency 7.5% and 1.38% in T1DM and controls respectively); rs57727346 (AC)₂₃/(AC)₂₅ (frequency 15.5% and 1.83% in T1DM and controls respectively); and rs10647945 (AC)₂₂/(AC)₂₅ (frequency 14.5% and 0.92% in T1DM and controls respectively) showed significant association with T1DM (Tables 6, 7, 8) and were considered to be T1DM risk genotypes. Finally, 62 T1DM patients (31%) were found to have risk genotypes at one locus (10 T1DM patients of the rs34580276 (TG)₁₉/(TG)₂₃; 22 T1DM patients of the rs57727346 (AC)₂₃/(AC)₂₅; and 21 T1DM patients of the rs10647945 (AC)₂₂/(AC)₂₅) or combination of risk genotypes at two or three loci (1 patient of the rs34580276//rs10647945; 4 patients of the rs57727346//rs10647945 and 4 patients of the rs34580276//rs57727346//rs10647945 risk genotypes). In control groups only 9 individuals were identified to have risk genotypes at one locus. Association analysis indicates on high T1DM susceptibility of the identified single locus risk genotypes and their combinations in risk multiple locus genotypes (Table 1). All detected associations supported by clinically significant levels of Odds Ratio (OR > 2; Table 1). All mentioned associated with T1DM genotypes and their combinations were considered to be T1DM risk factors.

**Table 1. Microsatellite genotypes associated with T1DM (T1DM risk markers)**

| Risk marker & risk genotype | Frequency in the groups' n (%) | | *P* OR [95% CI] |
|---|---|---|---|
| | T1DM n = 200 | Controls n = 218 | |
| rs34580276: | 10 (5.00) | 3 (1.38) | 3.89E-2 |
| (TG)₁₉/(TG)₂₃ | | | 3.633 [1.068 - 12.366] |
| rs57727346: | 22 (11.00) | 4 (1.83) | 2.75E-4 |
| (AC)₂₃/(AC)₂₅ | | | 5.995 [2.140 - 16.794] |
| rs10647945: | **21** (10.50) | **2** (0.92) | < 0.0001 |
| (AC)₂₂/(AC)₂₅ | | | 11.445 [3.046 - 42.999] |
| rs34580276 // rs57727346: | 1 (0.50) | 0 | - |
| (TG)₁₉/(TC)₂₃ // (AC)₂₃/(ACb | | | |
| rs57727346 // rs10647945: | 4 (2.00) | 0 | - |
| (AC)_{23/}(AC)₂₅ // (AC)₂₂/(AC)₂₅ | | | |
| rs34580276 // rs57727346 // rs10647945 : | 4 (2.00) | 0 | - |
| (TG)₁₉/(TG)₂₃(AC)₂₃(AC)₂₅//(AC)₂₂/(AC)₂₅ | | | |
| **Total** | **62 (31.00)** | **9 (4.13)** | < 0.0001 |
| | | | 5.087 [2.454 - 10.545] |

**Table 2. Detail information on the T1DM associated markers'**

| Marker ID¹ | HGVS² Names | Primer sequences | Fragment size (bp) / number of repeats |
|---|---|---|---|
| **rs34580276** (HSMS006, rs36054664, rs66507342) | *NC_000014.8: g.35784154_35784157delTGTG;* | F: 5'-CTAATTGACTTGCAGTTGCTGG-3' | 181/(TG)₁₈ |
| | *NG_011703.1:g.27581_27584delTGTG;* | R:5'-AACTGCCTCACAGGGCTG-3' | |
| | *NM_002791.1:c.683+493_683+496delTGTG;* | | |
| | *NT_026437.12:g.16784154_16784157delTGTG* | | |
| **rs57727346** (HSMS701, rs71928782, rs71862757, rs35123864 rs3223236, rs55940352, D14S1014) | *NC_000014.8:g.35620881_35620884delCACA;* | F: 5'-TCCATAGCACTTATCTGACATGC-3' | 136 / (AC)₂₅ |
| | *NM_001256678.1:c.1119+24064-1119+24067de...;* | R:5'-AAAATCCCTACCCTTGTGGTG-3' | |
| | *NM_001256679.1:c.882+24064 882+24067delC*..; | | |
| | *NM_001256680.1:c.51+24064-51+24067delCAC.*..; | | |
| | *NM_001256681.1:c.51+24064₋51+24067delCAC...;* | | |
| | *NM_014672.3:c.1167+24064_{_}1167+24067delCA...;* | | |
| | *NT_026437.12:g.16620881_16620884delCACA* | | |
| **rs10647945** (HSMS801, rs68081862, (rs68081863, rs71820751, rs72196957, rs60270535, *rs71444202)* | *NC_000014.8:g.35700153_35700154insCACACA..;* | F: 5'-ATGCAGTATGCCACCAACTGT-3' | 147 / (AC)₂₁ |
| | *NM_001256678.1:c.1228-35780_1228-35779in ...;* | R:5'-CCGAGAGGCAACCAGTATTATC-3' | |
| | *NM_001256679.1:c.991-35780_991-35779insC ..;* | | |
| | *NM_001256680.1:c.160-35780_160-35779insC ..;* | | |
| | *NM_001256681.1:c.160-35780_160-35779insC..;* | | |
| | *NM_014672.3:c.1276-35780_1276-35779insCA...;* | | |
| | *NT_026437.12:g.16700153_16700154insCACAC...* | | |

| | | | |
|---|---|---|---|
| ¹NCBI SNP database (www,ncbi.nhra.nih.gov) marker identification number, previously used and/or related names are given in brackets and illustrates in Figure 1, 2 and 3 for the rs34580276, rs57727346 and rs10647945 respectively; ²Human Genome variation and Variation of Society; HGVS (http://www.genomic.unimelb.edu.au/mdi/mutnomen/): Letters "F" and "R" in primer sequences indicate forward and reversal primers respectively | | | |

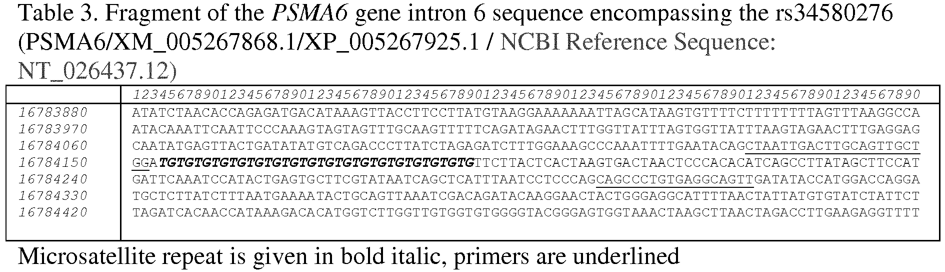

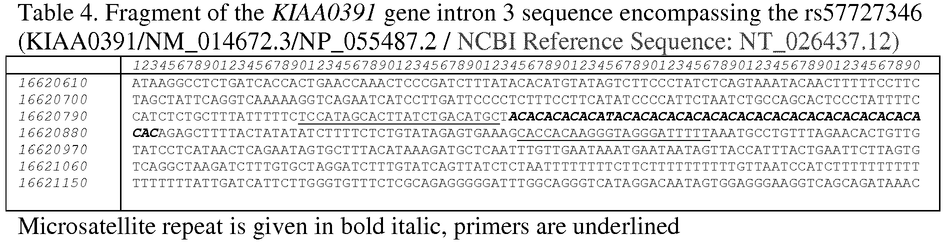

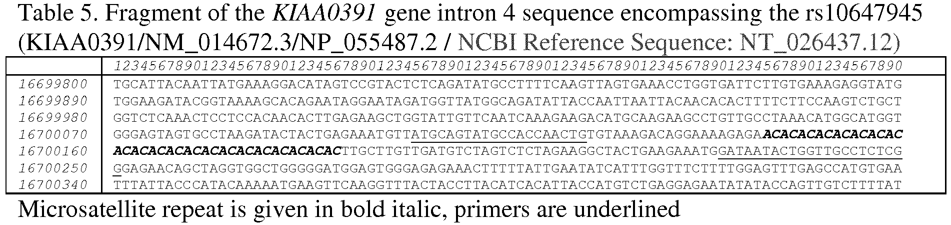

**Table 6. Presentation of the rs34580276 (HSMS006) genotypes in the groups of T1DM patients and healthy individuals .**

| Marker | Genotype | **Frequency in the groups. n (%)** | | | |
|---|---|---|---|---|---|
| | | Control (218) | | T1DM (200) | |
| | | n | % | n | % |
| **rs34580276 (HSMS006)** | (TG)₁₂/(TG)₂₁ | | | 1 | 0.5 |
| | (TG)₁₄/(TG)₂₁ | | | 1 | 0.5 |
| | (TG)₁₆/(TG)₂₂ | 2 | 0.92 | | |
| | (TG)₁₈/(TG)₁₈ | 24 | 11.01 | 6 | 3 |
| | (TG)₁₈/(TG)₁₉ | 13 | 5.96 | 17 | 8.5 |
| | (TG)₁₈/(TG)₂₀ | 1 | 0.46 | 6 | 3 |
| | (TG)₁₈/(TG)₂₁ | 39 | 17.89 | 23 | 11.5 |
| | (TG)₁₈/(TG)₂₂ | 9 | 4.13 | 22 | 11 |
| | (TG)₁₈/(TG)₂₃ | 3 | 1.38 | 7 | 3.5 |
| | (TG)₁₈/(TG)₂₄ | | | 4 | 2 |
| | (TG)₁₉/(TG)₁₉ | 30 | 13.76 | 8 | 4.0 |
| | (TG)₁₉/(TG)₂₀ | 3 | 1.38 | 3 | 1.5 |
| | (TG)₁₉/(TG)₂₁ | 17 | 7.80 | 16 | 8 |
| | (TG)₁₉/(TG)₂₂ | 11 | 5.05 | 13 | 6.5 |
| | (TG)₁₉/(TG)₂₃* | 3 | 1.38 | 15 | 7.5 |
| | (TG)₁₉/(TG)₂₄ | 1 | 0.46 | 2 | 1 |
| | (TG)₂₀/(TG)₂₀ | 1 | 0.46 | | 0 |
| | (TG)₂₀/(TG)₂₁ | 2 | 0.92 | 1 | 0.5 |
| | (TG)₂₀/(TG)₂₂ | 2 | 0.92 | 5 | 2.5 |
| | (TG)₂₀/(TG)₂₃ | 2 | 0.92 | 3 | 1.5 |
| | (TG)₂₀/(TG)₂₄ | 1 | 0.46 | 2 | 1.0 |
| | (TG)₂₁/(TG)₂₁ | 12 | 5.50 | 10 | 5 |
| | (TG)₂₁/(TG)₂₂ | 11 | 5.05 | 6 | 3 |
| | (TG)₂₁/(TG)₂₃ | 5 | 2.29 | 8 | 4 |
| | (TG)₂₁/(TG)₂₄ | 1 | 0.46 | 1 | 0.5 |
| | (TG)₂₁/(TG)₂₅ | 1 | 0.46 | | |
| | (TG)₂₂/(TG)₂₂ | 11 | 5.05 | 3 | 1.5 |
| | (TG)₂₂/(TG)₂₃ | 9 | 4.13 | 12 | 6 |
| | (TG)₂₂/(TG)₂₄ | 1 | 0.46 | 1 | 0.5 |
| | (TG)₂₃/(TG)₂₃ | 1 | 0.46 | 1 | 0.5 |
| | (TG)₂₃/(TG)₂₄ | 2 | 0.92 | 3 | 1.5 |

| | | | | | |
|---|---|---|---|---|---|
| *T1DM risk genotype: *P* < 0.05; OR = 5.450 95% CI [1.682 - 17.659] | | | | | |

**Table 7. Presentation of the rs57727346 (HSMS701) genotypes in the groups of T1DM patients and healthy individuals**

| Marker | Genotype | **Frequency in the groups. n (%)** | | | |
|---|---|---|---|---|---|
| | | Control (218) | | TIDM (200) | |
| | | n | % | n | % |
| **rs57727346 (HSMS701)** | (AC)₁₉/(AC)₂₄ | 1 | 0.46 | | |
| | (AC)₂₁/(AC)₂₁ | 5 | 2.29 | 1 | 0.50 |
| | (AC)₂₁/(AC)₂₂ | 5 | 2.29 | 6 | 3.00 |
| | (AC)₂₁/(AC)₂₃ | 5 | 2.29 | | |
| | (AC)₂₁/(AC)₂₄ | 12 | 5.50 | 2 | 1.00 |
| | (AC)₂₁/(AC)₂₅ | 12 | 5.50 | 1 | 0.50 |
| | (AC)₂₁/(AC)₂₆ | 7 | 3.21 | 1 | 0.50 |
| | (AC)₂₁/(AC)₂₇ | 1 | 0.46 | | |
| | (AC)₂₂/(AC)₂₂ | 25 | 11.47 | 11 | 5.50 |
| | (AC)₂₂/(AC)₂₃ | 5 | 2.29 | 5 | 2.50 |
| | (AC)₂₂/(AC)₂₄ | 10 | 4.59 | 6 | 3.00 |
| | (AC)₂₂/(AC)₂₅ | 19 | 8.72 | 20 | 10.00 |
| | (AC)₂₂/(AC)₂₆ | 12 | 5.50 | 12 | 6.00 |
| | (AC)₂₃/(AC)₂₃ | 11 | 5.05 | 8 | 4.00 |
| | (AC)₂₃/(AC)₂₄ | 2 | 0.92 | 6 | 3.00 |
| | (AC)₂₃/(AC)₂₅* | 4 | 1.83 | 31 | 15.50 |
| | (AC)₂₃/(AC)₂₆ | 11 | 5.05 | 13 | 6.50 |
| | (AC)₂₃/(AC)₂₇ | | 0.00 | 2 | 1.00 |
| | (AC)₂₄/(AC)₂₄ | 15 | 6.88 | 1 | 0.50 |
| | (AC)₂₄/(AC)₂₅ | 4 | 1.83 | 7 | 3.50 |
| | (AC)₂₄/(AC)₂₆ | 5 | 2.29 | 5 | 2.50 |
| | (AC)₂₄/(AC)₂₇ | 3 | 1.38 | | |
| | (AC)₂₄/(AC)₂₉ | | | 1 | 0.50 |
| | (AC)₂₅/(AC)₂₅ | 24 | 11.01 | 35 | 17.50 |
| | (AC)₂₅/(AC)₂₆ | 10 | 4.59 | 17 | 8.50 |
| | (AC)₂₅/(AC)₂₇ | 1 | 0.46 | 2 | 1.00 |
| | (AC)₂₅/(AC)₂₈ | 1 | 0.46 | | |
| | (AC)₂₆/(AC)₂₆ | 6 | 2.75 | 7 | 3.50 |
| | (AC)₂₆/(AC)₂₇ | 2 | 0.92 | | |

| | | | | | |
|---|---|---|---|---|---|
| *TIDM risk genotype: *P* < 0.0001; OR = 8.448 95°7o CI [3.089 - 23.104] | | | | | |

**Table 8. Presentation of the rs10647945) (HSMS801) genotypes in the groups of T1DM patients and healthy individuals**

| Marker | Genotype | **Frequency in the groups. n (%)** | | | |
|---|---|---|---|---|---|
| | | Control (218) | | T1DM (200) | |
| | | n | % | n | % |
| **rs10647945 HSMS801** | (AC)₁₁/(AC)₁₅ | 1 | 0.46 | 1 | 0.50 |
| | (AC)₁₄/(AC)₁₄ | 1 | 0.46 | | |
| | (AC)₁₅/(AC)₁₅ | 3 | 1.38 | | |
| | (AC)₁₅/(AC)₁₇ | 5 | 2.29 | 5 | 2.50 |
| | (AC)₁₅/(AC)₁₉ | 1 | 0.46 | | |
| | (AC)₁₅/(AC)₂₀ | 1 | 0.46 | 3 | 1.50 |
| | (AC)₁₅/(AC)₂₁ | 6 | 2.75 | 4 | 2.00 |
| | (AC)₁₅/(AC)₂₂ | 4 | 1.83 | 6 | 3.00 |
| | (AC)₁₅/(AC)₂₃ | 2 | 0.92 | | |
| | (AC)₁₅/(AC)₂₄ | | | 2 | 1.00 |
| | (AC)₁₅/(AC)₂₅ | 2 | 0.92 | | |
| | (AC)₁₅/(AC)₂₇ | 1 | 0.46 | | |
| | (AC)₁₆/(AC)₁₇ | 1 | 0.46 | | |
| | (AC)₁₆/(AC)₂₀ | 3 | 1.38 | | |
| | (AC)₁₆/(AC)₂₂ | | | 5 | 2.50 |
| | (AC)₁₇/(AC)₁₇ | 11 | 5.05 | 3 | 1.50 |
| | (AC)₁₇/(AC)₁₉ | 1 | 0.46 | 4 | 2.00 |
| | (AC)₁₇/(AC)₂₀ | 4 | 1.83 | 7 | 3.50 |
| | (AC)₁₇/(AC)₂₁ | 20 | 9.17 | 19 | 9.50 |
| | (AC)₁₇/(AC)₂₂ | 7 | 3.21 | 7 | 3.50 |
| | (AC)₁₇/(AC)₂₃ | 8 | 3.67 | | |
| | (AC)₁₇/(AC)₂₄ | | | 1 | 0.50 |
| | (AC)₁₇/(AC)₂₅ | 4 | 1.83 | 2 | 1.00 |
| | (AC)₁₇/(AC)₂₇ | | | 1 | 0.50 |
| | (AC)₁₈/(AC)₂₀ | | | 1 | 0.50 |
| | (AC)₁₈/(AC)₂₁ | 1 | 0.46 | 3 | 1.50 |
| | (AC)₁₈/(AC)₂₂ | | | 3 | 1.50 |
| | (AC)l₈/(AC)₂₄ | | | 1 | 0.50 |
| | (AC)₁₉/(AC)₂₁ | 2 | 0.92 | 2 | 1.00 |
| | (AC)₁₉/(AC)₂₂ | 3 | 1.38 | 4 | 2.00 |
| | (AC)₁₉/(AC)₂₃ | 1 | 0.46 | | |
| | (AC)₁₉/(AC)₂₇ | 1 | 0.46 | | |
| | (AC)₂₀/(AC)₂₀ | 2 | 0.92 | 1 | 0.50 |
| | (AC)₂₀/(AC)₂₁ | 15 | 6.88 | 14 | 7.00 |
| | (AC)₂₀/(AC)₂₂ | 4 | 1.83 | 3 | 1.50 |
| | (AC)₂₀/(AC)₂₃ | 2 | 0.92 | 1 | 0.50 |
| | (AC)₂₀/(AC)₂₄ | 2 | 0.92 | 2 | 1.00 |
| | (AC)₂₀/(AC)₂₅ | 4 | 1.83 | 1 | 0.50 |
| | (AC)₂₀/(AC)₂₆ | 4 | 1.83 | | |
| | (AC)₂₁/(AC)₂₁ | 22 | 10.09 | 13 | 6.50 |
| | (AC)₂₁/(AC)₂₂ | 15 | 6.88 | 19 | 9.50 |
| | (AC)₂₁/(AC)₂₃ | 6 | 2.75 | 1 | 0.50 |
| | (AC)₂₁/(AC)₂₄ | 2 | 0.92 | 8 | 4.00 |
| | (AC)₂₁/(AC)₂₅ | 9 | 4.13 | 7 | 3.50 |
| | (AC)₂₁/(AC)₂₆ | 4 | 1.83 | 3 | 1.50 |
| | (AC)₂₁/(AC)₂₇ | 3 | 1.38 | 1 | 0.50 |
| | (AC)₂₁/(AC)₂₈ | 1 | 0.46 | | |
| | (AC)₂₂/(AC)₂₂ | 13 | 5.96 | 5 | 2.50 |
| | (AC)₂₂/(AC)₂₅* | 2 | 0.92 | 29 | 14.50 |
| | (AC)₂₂/(AC)₂₇ | 1 | 0.46 | 1 | 0.50 |
| | (AC)₂₃/(AC)₂₃ | 4 | 1.83 | 2 | 1.00 |
| | (AC)₂₃/(AC)₂₄ | | | 3 | 1.50 |
| | (AC)₂₃/(AC)₂₅ | 2 | 0.92 | | |
| | (AC)₂₃/(AC)₂₆ | 1 | 0.46 | | |
| | (AC)₂₄/(AC)₂₄ | 2 | 0.92 | | |
| | (AC)₂₄/(AC)₂₅ | 1 | 0.46 | | |
| | (AC)₂₅/(AC)₂₅ | 2 | 0.92 | 2 | 1.00 |
| | (AC)₂₇/(AC)₂₇ | 1 | 0.46 | | |

| | | | | | |
|---|---|---|---|---|---|
| *T1DM risk genotype: *P* < 0.0001; OR = 15.805 95% CI [4.285 - 58.295] | | | | | |

### References

1. Abhary S, Hewitt AW, Burdon KP, Craig JE. A systematic meta-analysis of genetic association studies for diabetic retinopathy. Diabetes. 2009 Sep;58(9):2137-47.
2. Aghdam SY, Gurel Z, Ghaffarieh A, Sorenson CM, Sheibani N.High glucose and diabetes modulate cellular proteasome function: Implications in the pathogenesis of diabetes complications. Biochem Biophys Res Commun. 2013 Mar 8;432(2):339-44
3. Akhter Q, Masood A, Ashraf R, Majid S, Rasool S, Khan T, Rashid T, Sameer AS, Ganai BA. Polymorphisms in the 3'UTR of the human leptin gene and their role in hypertension. Mol Med Rep. 2012 Apr;5(4):1058-62.
4. Alsmadi O, Muiya P, Khalak H, A1-Saud H, Meyer BF, A1-Mohanna F, Alshahid M, Dzimiri N. Haplotypes encompassing the KIAA0391 and PSMA6 gene cluster confer a genetic link for myocardial infarction and coronary artery disease. Ann Hum Genet. 2009 Sep;73(Pt 5):475-83.
5. Bachmann HS, Novotny J, Sixt S, Liebisch P, Frey UH, Dührsen U, Siffert W, Nückel H. The G-Allele of the PSMA6-8C>G polymorphism is associated with poor outcome in multiple myeloma independently of circulating proteasome serum levels. Eur J Haematol. 2010 Aug;85(2):108-13.
6. Bao W, Song F, Li X, Rong S, Yang W, Wang D, Xu J, Fu J, Zhao Y, Liu L. Association between heme oxygenase-1 gene promoter polymorphisms and type 2 diabetes mellitus: a HuGE review and meta-analysis. Am J Epidemiol. 2010 Sep 15;172(6):631-6.
7. Barbieri M, Marfella R, Rizzo MR, Boccardi V, Siniscalchi M, Schiattarella C, Siciliano S, Lemme P, Paolisso G. The -8 UTR C/G polymorphism of PSMA6 gene is associated with susceptibility to myocardial infarction in type 2 diabetic patients. Atherosclerosis. 2008 Nov;201(1):117-23.
8. Bassuny WM, Ihara K, Sasaki Y, Kuromaru R, Kohno H, Matsuura, N, Hara T, 2003. A functional polymorphism in the promoter/enhancer region of the FOXP3/Scurfin gene associated with type 1 diabetes. Immunogenetics 55:149-156.
9. Ben Mustapha M, Moumni I, Zorai A, Douzi K, Ghanem A, Abbes S. Microsatellite and single nucleotide polymorphisms in the β-globin locus control region-hypersensitive Site 2: SPECIFICITY of Tunisian βs chromosomes. Hemoglobin. 2012;36(6):533-44.
10. Bennett DA, Xu P, Clarke R, Zondervan K, Parish S, Palmer A, Cardon L, Peto R, Lathrop M, Collins R; International Study of Infarct Survival Collaborators. The exon 1-8C/G SNP in the PSMA6 gene contributes only a small amount to the burden of myocardial infarction in 6946 cases and 2720 controls from a United Kingdom population. Eur J Hum Genet. 2008 Apr;16(4):480-6.
11. Bidichandani SI, Ashizawa T, Patel PI. The GAA triplet-repeat expansion in Friedreich ataxia interferes with transcription and may be associated with an unusual DNA structure. Am J Hum Genet. 1998;62(1):111-21.
12. Boulikas T, 1993. Nature of DNA sequences at the attachment regions of genes to the nuclear matrix. J Cell Biochem 52: 14-22.
13. Chen HY, Huang W, Leung VH, Fung SL, Ma SL, Jiang H, Tang NL. Functional interaction between SNPs and microsatellite in the transcriptional regulation of insulin-like growth factor 1. Hum Mutat. 2013a Sep;34(9):1289-97.
14. Chen YH, Hung SC, Tarng DC. Length polymorphism in heme oxygenase-1 and cardiovascular events and mortality in hemodialysis patients. Clin J Am Soc Nephrol. 2013b Oct;B(10):1756-63.
15. Chin JY, Schleifman EB, Glazer PM, 2007. Repair and recombination induced by triple helix DNA. Front Biosci 12: 4288-4297.
16. Filippova GN, Thienes CP, Penn BH, Cho DH, Hu YJ, Moore JM, Klesert TR, Lobanenkov VV, Tapscott SJ, 2001. CTCF-binding sites flank CTG/CAG repeats and form a methylation-sensitive insulator at the DM1 locus. Nature Genet 28: 335-343.
17. Freudenburg W, Gautam M, Chakraborty P, James J, Richards J, Salvatori AS, Baldwin A, Schriewer J, Buller RM, Corbett JA, Skowyra D. Immunoproteasome Activation During Early Antiviral Response in Mouse Pancreatic β-cells: New Insights into Auto-antigen Generation in Type I Diabetes?. J Clin Cell Immunol. 2013 Apr 23;4(2). pii: 141.
18. Gao C, Chen G, Liu L, Li X, He J, Jiang L, Zhu J, Xu Y. Impact of high glucose and proteasome inhibitor MG132 on histone H2A and H2B ubiquitination in rat glomerular mesangial cells. J Diabetes Res. 2013;2013:589474.
19. Grandi FC, An W. Non-LTR retrotransposons and microsatellites: Partners in genomic variation.. Mob Genet Elements. 2013 Jul 1;3(4):e25674.
20. Gregorek AC, Gornik KC, Polancec DS, Dabelic S. GT microsatellite repeats in the heme oxygenase-1 gene promoter associated with abdominal aortic aneurysm in Croatian patients. Biochem Genet. 2013 Jun;51(5-6):482-92.
21. Gulcher J. Microsatellite markers for linkage and association studies. Cold Spring Harb Protoc. 2012 Apr 1;2012(4):425-32.
22. Heckman MG, Soto-Ortolaza AI, Diehl NN, Rayaprolu S, Brott TG, Wszolek ZK, Meschia JF, Ross OA. Genetic variants associated with myocardial infarction in the PSMA6 gene and Chr9p21 are also associated with ischaemic stroke. Eur J Neurol. 2013 Feb;20(2):300-8.
23. Hinohara K, Nakajima T, Sasaoka T, Sawabe M, Lee BS, Ban J, Park JE, Izumi T, Kimura A. Replication studies for the association of PSMA6 polymorphism with coronary artery disease in East Asian populations. J Hum Genet. 2009 Apr;54(4):248-51.
24. Hofmeister-Brix A, Lenzen S, Baltrusch S. The ubiquitin proteasome system regulates the stability and activity of the glucose sensor glucokinase in pancreatic beta cells. Biochem J. 2013 Sep 12. [Epub ahead of print]
25. Hsueh WC, St Jean PL, Mitchell BD, Pollin TI, Knowler WC, Ehm MG, Bell CJ, Sakul H, Wagner MJ, Burns DK, Shuldiner AR. Genome-wide and fine-mapping linkage studies of type 2 diabetes and glucose traits in the Old Order Amish: evidence for a new diabetes locus on chromosome 14q11 and confirmation of a locus on chromosome 1q21-q24. Diabetes. 2003 Feb;52(2):550-7.
26. Hu YF, Lee KT, Wang HH, Ueng KC, Yeh HI, Chao TF, Liao JN, Lin YJ, Chang SL, Lo LW, Tuan TC, Li CH, Chung FP, Hsu CP, Chang HH, Huang CH, Chen SA. The association between heme oxygenase-1 gene promoter polymorphism and the outcomes of catheter ablation of atrial fibrillation. PLoS One. 2013;8(2):e56440.
27. Hui J, Hung LH, Heiner M, Schreiner S, Neumüller N, Reither G, Haas SA, Bindereif A, 2005. Intronic CA-repeat and CA-rich elements: a new class of regulators of mammalian alternative splicing. EMBO J 24: 1988-1998.
28. Ikeda S, Tanaka N, Arai T, Chida K, Muramatsu M, Sawabe M. Polymorphisms of LTA, LGALS2, and PSMA6 genes and coronary atherosclerosis: a pathological study of 1503 consecutive autopsy cases. Atherosclerosis. 2012 Apr;221(2):458-60.
29. Jarne P, Lagoda PJ. Microsatellites, from molecules to populations and back. Trends Ecol Evol. 1996 Oct;11(10):424-9.
30. Jemaa R, Ben Ali S, Kallel A, Feki M, Elasmi M, Taieb SH, Sanhaji H, Omar S, Kaabachi N. Association of a 27-bp repeat polymorphism in intron 4 of endothelial constitutive nitric oxide synthase gene with hypertension in a Tunisian population. Clin Biochem. 2009 Jun;42(9):852-6.
31. Kim JC, Mirkin SM. The balancing act of DNA repeat expansions.. Curr Opin Genet Dev. 2013 Jun;23(3):280-8.
32. King DG. Evolution of simple sequence repeats as mutable sites. Adv Exp Med Biol. 2012;769:10-25.
33. P.M. Kloetzel "Antigen processing by the proteasome," Nat Rev Mol Cell Biol, vol. 2, pp. 179-187, 2001.
34. Lee JH, Jeon MH, Seo YJ, Lee YJ, Ko JH, Tsujimoto Y, Lee JH, 2004. CA repeats in the 3'-untranslated region of bcl-2 mRNA mediate constitutive decay of bcl-2 mRNA. J Biol Chem 279: 42758-42764.
35. Lenartowski R, Goc A, 2002. Tissue-specific association of the human tyrosine hydroxylase gene with the nuclear matrix. Neurosci Lettr 330: 151-154.
36. Li G, Tolstonog GV, Traub P, 2003. Interaction in vitro of type III intermediate filament proteins with Z-DNA and B-Z-DNA junctions. DNA Cell Biol 22: 141-169.
37. Li Y, Seidel K, Marschall P, Klein M, Hope A, Schacherl J, Schmitz J, Menk M, Schefe JH, Reinemund J, Hugel R, Walden P, Schlosser A, Volkmer R, Schimkus J, Kölsch H, Maier W, Kornhuber J, Frölich L, Klare S, Kirsch S, Schmerbach K, Scheele S, Grittner U, Zollmann F, Goldin-Lang P, Peters O, Kintscher U, Unger T, Funke-Kaiser H. A polymorphic microsatellite repeat within the ECE-1c promoter is involved in transcriptional start site determination, human evolution, and Alzheimer's disease. J Neurosci. 2012 Nov 21;32(47):16807-20.
38. Liu X, Wang X, Shen Y, Wu L, Ruan X, Lindpaintner K, Yusuf S, Engert JC, Anand S, Tan X, Liu L. The functional variant rs1048990 in PSMA6 is associated with susceptibility to myocardial infarction in a Chinese population. Atherosclerosis. 2009 Sep;206(1):199-203.
39. Liu J, Yuan XJ, Liu JX, Tian LM, Quan JX, Liu J, Chen XH, Wang YF, Shi ZY, Zhang JL. Validation of the association between PSMA6 -8 C/G polymorphism and type 2 diabetes mellitus in Chinese Dongxiang and Han populations. Diabetes Res Clin Pract. 2012 Nov;98(2):295-301.
40. Merforth, S., Kuehn L, Osmers, A., Dahlmann, B. (2003) Alteration of 20S proteasome subtypes and proteasome activator PA28 in skeletal muscle of rat after induction of diabetes mellitus. Int. J. Biochem. Cell. Biol. 35, 740-748.
41. Michalova E, Vojtesek B, Hrstka R. Impaired pre-mRNA processing and altered architecture of 3' untranslated regions contribute to the development of human disorders. Int J Mol Sci. 2013 Jul 26;14(8):15681-94.
42. Mune T, Suwa T, Morita H, Isomura Y, Takada N, Yamamoto Y, Hayashi M, Yamakita N, Sasaki A, Takeda N, Takeda J, White PC, Kaku K. Longer HSD11B2 CA-repeat in impaired glucose tolerance and type 2 diabetes. Endocr J. 2013;60(5):671-8.
43. Nokoff N, Rewers M. Pathogenesis of type 1 diabetes: lessons from natural history studies of high-risk individuals. Ann N Y Acad Sci. 2013 Apr;1281:1-15.
44. Oliveira-Paula GH, Lacchini R, Coeli-Lacchini FB, Junior HM, Tanus-Santos JE. Inducible nitric oxide synthase haplotype associated with hypertension and responsiveness to antihypertensive drug therapy. Gene. 2013 Feb 25;515(2):391-5.
45. Permutt, M.A., Wasson, J.C., Suarez, B.K., Lin, J., Thomas, J., Meyer, J., Lewitzky, S., Rennich, J.S., Parker, A., DuPrat, L., Maruti, S., Chayen, S., Glaser, B. (2001) A genome scan for type 2 diabetes susceptibility loci in a genetically isolated population. Diabetes. 50, 681-685
46. Petrovic, J., Hall, H., Mehr, R., Glas, R., Hoglund, P. (2004) Inhibition of the proteasome reduces transfer-induced diabetes in nonobese diabetic mice. Scand. J. Immunol. 60, 134-142.
47. Rockman MV, Wray GA. Abundant raw material for cis-regulatory evolution in humans. Mol Biol Evol. 2002 Nov;19(11):1991-2004.
48. Rothenburg S, Koch-Nolte F, Haag F, 2001. DNA methylation and Z-DNA formation as mediators of quantitative differences in the expression of alleles. Immunol Rev 184: 286-298.
49. Sawaya S, Bagshaw A, Buschiazzo E, Kumar P, Chowdhury S, Black MA, Gemmell N. Microsatellite tandem repeats are abundant in human promoters and are associated with regulatory elements. PLoS One. 2013;8(2):e54710.
50. Sharma AK, Khanna D. Diabetes mellitus associated cardiovascular signaling alteration: a need for the revisit. Cell Signal. 2013 May;25(5):1149-55
51. Silander K, Scott LJ, Valle TT, Mohlke KL, Stringham HM, Wiles KR, Duren WL, Doheny KF, Pugh EW, Chines P, Narisu N, White PP, Fingerlin TE, Jackson AU, Li C, Ghosh S, Magnuson VL, Colby K, Erdos MR, Hill JE, Hollstein P, Humphreys KM, Kasad RA, Lambert J, Lazaridis KN, Lin G, Morales-Mena A, Patzkowski K, Pfahl C, Porter R, Rha D, Segal L, Suh YD, Tovar J, Unni A, Welch C, Douglas JA, Epstein MP, Hauser ER, Hagopian W, Buchanan TA, Watanabe RM, Bergman RN, Tuomilehto J, Collins FS, Boehnke M. A large set of Finnish affected sibling pair families with type 2 diabetes suggests susceptibility loci on chromosomes 6, 11, and 14. Diabetes. 2004 Mar;53(3):821-9.
52. Sjakste T, Zhuk A, 2006. Novel haplotype description and structural background of the eventual functional significance of the barley beta-amylase gene intron rearrangements. Theor Appl Genet 113: 1063-1079.
53. Sjakste T, Eglite J, Sochnevs A, Marga M, Pirags V, Collan Y, Sjakste N. Microsatellite genotyping of chromosome 14q13.2-14q13 in the vicinity of proteasomal gene PSMA6 and association with Graves' disease in the Latvian population. Immunogenetics 2004 56(4):238-43.
54. Sjakste T., Kalis M., Poudziunas I., Pirags V., Lazdins M., Groop L., Sjakste N. Association of microsatellite polymorphisms of the human 14q13.2 region with type 2 diabetes mellitus in Latvian and Finnish populations. Ann Human Genet 2007a 71:772-776.
55. Sjakste T, Lauberte L, Collan Y, Savontaus ML, Bajare A, Scherrer K, Sjakste N. Identification of an intronic TG repeat polymorphism in the human proteasome core particle PROS-27K gene. DNA Seq 2002 13(3): 139-43.
56. Sjakste T., Poudziunas I., Ninio E., Perret C., Pirags V., Nicaud V., Lazdins M., Evans A., Morrison C., Cambien F., Sjakste N. SNPs of PSMA6 gene - investigation of possible association with myocardial infarction and type 2 diabetes mellitus. Russ J Genet 2007b 43:442-448.
57. Sjakste T., Poudziunas I., Pirags V., Lazdins M., N. Sjakste Bioinformatical analysis of evolutional conservatism and functional significance of microsatellite alleles of human 14q13.2 region associated with type 2 diabetes mellitus. Proc. Latv. Acad. Sci. B 2008 62(3):91-102.
58. Sjakste T, Sjakste N, Scherrer K. Exon/intron organisation of human proteasome PROS-27 K gene. DNA Seq 2001 12(4):261-5.
59. Sjakste T, Trapina I, Rumba-Rozenfelde I, Lunin R, Sugoka O, Sjakste N. Identification of a Novel Candidate Locus for Juvenile Idiopathic Arthritis at 14q13.2 in the Latvian Population by Association Analysis with Microsatellite Markers. DNA Cell Biol. 2010 29:543-551.
60. Song F, Li X, Zhang M, Yao P, Yang N, Sun X, Hu FB, Liu L. Association between heme oxygenase-1 gene promoter polymorphisms and type 2 diabetes in a Chinese population. Am J Epidemiol. 2009 Sep 15;170(6):747-56.
61. Stanworth RD, Kapoor D, Channer KS, Jones TH. Dyslipidaemia is associated with testosterone, oestradiol and androgen receptor CAG repeat polymorphism in men with type 2 diabetes. Clin Endocrinol (Oxf). 2011 May;74(5):624-30.
62. Sun JX, Helgason A, Masson G, Ebenesersdóttir SS, Li H, Mallick S, Gnerre S, Patterson N, Kong A, Reich D, Stefansson K. A direct characterization of human mutation based on microsatellites. Nat Genet. 2012 Oct;44(10):1161-5.
63. T. Tanaka, Y. Nakamura, A. Iida, K. Ozaki, M. Hori Patent US8158351 - Method for determining inflammatory disease. Patent 8158351 Issued on April 17, 2012.
64. Tautz D., Schlötterer C. (1994). "Simple sequences". Current Opinion in Genetics & Development 4 (6): 832-837.
65. Tolstonog GV, Li G, Shoeman RL, Traub P, 2005. Interaction in vitro of type III intermediate filament proteins with higher order structures of single-stranded DNA, particularly with G-quadruplex DNA. DNA Cell Biol 24: 85-110.
66. Turnpenny, P. & Ellard, S. (2005). Emery's Elements of Medical Genetics, 12th. ed. Elsevier, Lond*on.*
67. Van Wormhoudt A, Sellos D, 2003. Highly variable polymorphism of the alpha-amylase gene family in Litopenaeus vannamei (Crustacea Decapoda). J Mol Evol 57: 659-671.
68. Wang H, Jiang M, Zhu H, Chen Q, Gong P, Lin J, Lu J, Qiu J. Quantitative assessment of the influence of PSMA6 variant (rs1048990) on coronary artery disease risk. Mol Biol Rep. 2013 Feb;40(2):1035-41.
69. Wang G, Vasquez KM, 2007. Z-DNA, an active element in the genome. Front Biosci 12: 4424-4438.
70. Weber JL, Wong C. Mutation of human short tandem repeats. Hum Mol Genet. 1993 Aug;2(8):1123-8.
71. Wu ML, Ho YC, Yet SF. A central role of heme oxygenase-1 in cardiovascular protection. Antioxid Redox Signal. 2011 Oct 1;15(7):1835-46.
72. Workeneh B, Bajaj M. The regulation of muscle protein turnover in diabetes. Int J Biochem Cell Biol. 2013 Oct;45(10):2239-44.
73. Zhu JM, Wang B, Li J, Chen GM, Fan YG, Feng CC, Pan HF, Ye DQ. D18S880 microsatellite polymorphism of carnosinase gene and diabetic nephropathy: a meta-analysis. Genet Test Mol Biomarkers. 2013 Apr;17(4):289-94.

## Claims

1. A method for *in vitro* determining that a human subject has or is at risk of developing type 1 *diabetes mellitus,* said method comprising: detecting, in a genomic DNA present in an isolated sample of biological material removed from the subject, the rs71928782 locus heterozygote genotype formed by (TG)₁₉ (SEQ ID No 1) and (TG)₂₃ (SEQ ID No 2), or the rs57727346 locus heterozygote genotype formed by (AC)₂₃ (SEQ ID No 3) and (AC)₂₅ (SEQ ID No 4), or the rs 10647945 heterozygote genotype formed by (AC)₂₂ (SEQ ID No 5) and (AC)₂₅ (SEQ ID No 6) or their combinations, wherein presence of said genotypes indicates the presence of, or a risk of developing, type 1 *diabetes mellitus* disease in the subject.

2. A kit suitable for the detecting, diagnosing, prediction, prognosis, staging or monitoring type 1 *diabetes mellitus,* comprising DNA amplified fragment having nucleotide sequence SEQ ID No. 1 and DNA amplified fragment having nucleotide sequence SEQ ID No. 2, and/or DNA amplified fragment having nucleotide sequence SEQ ID No 3 and DNA amplified fragment having nucleotide sequence SEQ ID No 4, and/or DNA amplified fragment having nucleotide sequence SEQ ID No 5 and DNA amplified fragment having nucleotide sequence SEQ ID No 6.

3. The kit according to claim 2, comprising synthetic primers of sequences SEQ ID No. 7 and SEQ ID No. 8, and/or SEQ ID No. 9 and SEQ ID No. 10, and/or SEQ ID No. 11 and SEQ ID No. 12.

4. Use of the kit according to claim 2 or 3 for the detecting, prediction, diagnosis, prognosis, staging or monitoring type 1 *diabetes mellitus.*
